# EUROPÄISCHE PATENTSCHRIFT

(11) **EP 0 940 389 B1**
(45) Veröffentlichungstag und Bekanntmachung des Hinweises auf die Patenterteilung: **28.05.2003**
(21) Anmeldenummer: 99104113.8
(22) Anmeldetag: 02.03.1999
(51) Int. Cl.: C07C 267/00, C07C 271/10, C07C 275/04

(54) **Carbodiimide und Verfahren zu deren Herstellung**
Carbodiimides and process for their preparation
Carbodiimides et procédé de leur préparation

(30) Priorität: 06.03.1998 DE 19809634
(43) Veröffentlichungstag der Anmeldung: 08.09.1999
(73) Patentinhaber: BASF AKTIENGESELLSCHAFT, 67056 Ludwigshafen (DE)
(72) Erfinder: Kokel, Nicolas Dr., 13980 Alleins (FR); Häberle, Karl Dr., 67346 Speyer (DE); Kraus, Rupert Dr., 67165 Waldsee (DE)

(56) Entgegenhaltungen:
- EP-A- 0 628 541
- DE-A- 4 442 724
- US-A- 5 210 170
- US-A- 5 614 483

## Beschreibung

Die Erfindung betrifft Verfahren zur Herstellung von Carbodiimiden, derart ähnliche Carbodiimide und Mischungen, enthaltend diese Carbodiimide und Verbindungen, die Esterstrukturen aufweisen, bevorzugt Polyurethane, die Esterstrukturen aufweisen.

Organische Carbodiimide sind bekannt und finden beispielsweise Verwendung als Stabilisator gegen den hydrolytischen Abbau von Estergruppen enthaltenden Verbindungen, beispielsweise Polyadditions- und Polykondensationsprodukten wie z.B. Polyurethanen. Carbodiimide können nach allgemein bekannten Verfahren hergestellt werden, beispielsweise durch Einwirkung von Katalysatoren auf Mono- oder Polyisocyanate unter Kohlendioxidabspaltung. Als Katalysatoren geeignet sind z.B. heterocyclische, Phosphor gebunden enthaltende Verbindungen, Metallcarbonyle, Phospholine, Phospholene und Phospholidine sowie deren Oxide und Sulfide.

Derartige Carbodiimide, ihre Herstellung und deren Verwendung als Stabilisatoren gegen die hydrolytische Spaltung von Kunststoffen auf Polyesterbasis werden z.B. beschrieben in DE-A 4 318 979, DE-A 4 442 724 und EP-A 460 481.

DE-A 4 318 979 beschreibt die Verwendung von 1,3-Bis-(1-methyl-1-isocyanato-ethyl)-benzol zur Herstellung von Carbodiimiden und/oder oligomeren Polycarbodiimiden mit endständigen Isocyanat-, Harnstoff- und/oder Urethangruppen. Die endständigen Isocyanatgruppen der in dieser Schrift beschriebenen Carbodiimide werden üblicherweise mit Monoalkoholen oder Monoaminen umgesetzt. Nachteilig an diesen Carbodiimiden ist die Tatsache, daß es bei diesen Verbindungen um viskose Flüssigkeiten handelt, die in den in der kunststoffverarbeitenden Industrie üblichen, auf die Handhabung von pulverförmigen oder granulierten Feststoffen ausgelegten Dosieranlagen schwierig zu verarbeiten sind.

EP-A 460 481 offenbart substituierte Monocarbodiimide oder oligomere, substituierte Polycarbodiimide mit bis zu vier Carbodiimidgruppen, die hergestellt werden aus substituierten Diisocyanaten und die praktisch weder in der Hitze, z.B. unter den üblichen Verarbeitungsbedingungen, noch bei Raumtemperatur giftige, flüchtige aus den eingesetzten Carbodiimiden stammende Substanzen abspalten. Diese Carbodiimide weisen den erheblichen Nachteil auf, daß die Carbodiimidgruppe direkt an aromatische Kohlenstoffatome gebunden ist. Eine Spaltung der Carbodiimidgruppe, z.B. unter verschärften hydrolytischen Bedingungen, führt in diesem Fall zu aromatischen Aminen, die hinsichtlich ihrer Toxizität nicht unbedenklich sind. Zudem weisen die in EP-A 460 481 beschriebenen Carbodiimide eine vergleichsweise große Tendenz zur Vergilbung auf, was bei vielfältigen Anwendungen unerwünscht ist.

Die Aufgabe der vorliegenden Erfindung bestand somit darin, Carbodiimide als Stabilisatoren gegen die hydrolytische Spaltung von Kunststoffen auf Polyesterbasis zu entwickeln, die als Feststoff einfacher mit den Kunststoffen gemischt werden können und hervorragende Eigenschaften als Stabilisatoren aufweisen.

Diese Aufgabe konnte überraschenderweise gelöst werden durch ein Verfahren zur Herstellung von bei Raumtemperatur festen Carbodiimiden enthaltend Carbodiimidstrukturen sowie Urethan- und/oder Harnstoffstrukturen, wobei 1,3-Bis-(1-methyl-1-isocyanatoethyl)benzol, dessen Isocyanatgruppen nicht an aromatische Kohlenstoffatome gebunden sind, in Gegenwart von Katalysatoren unter Kohlendioxidabspaltung zu Carbodiimiden umsetzt und anschließend das Isocyanatgruppen-aufweisende Carbodiimid mit Verbindungen, die mindestens zwei gegenüber Isocyanaten reaktive Gruppen aufweisen, umsetzt..

Die Herstellung der erfindungsgemäßen Carbodiimide erfolgt im wesentlichen durch zwei Umsetzungsschritte. Zum einen werden (1) durch allgemein bekannte Umsetzung der Isocyanatgruppen miteinander unter Abspaltung von Kohlendioxid in Gegenwart von üblichen Katalysatoren, die für diese Umsetzung bekannt sind und eingangs beschrieben wurden, Carbodiimidstrukturen erzeugt, zum anderen werden (2) Isocyanatgruppen mit gegenüber Isocyanaten reaktiven Verbindungen zur Herstellung von Urethan- und/oder Harnstoffstrukturen umgesetzt. Die Reihenfolge dieser beiden wesentlichen Verfahrensschritte (1) und (2) ist beliebig, soweit für die jeweilige Umsetzung freie Isocyanatgruppen vorliegen.

Beispielsweise kann man die erfindungsgemäßen Carbodiimide derart erhalten, daß man 1,3-Bis-(1-methyl-1-isocyanato-ethyl)benzol, dessen Isocyanatgruppen nicht an aromatische Kohlenstoffatome gebunden sind, in Gegenwart von Katalysatoren unter Kohlendioxidfreisetzung zu Carbodiimiden umsetzt und anschließend das Isocyanatgruppen aufweisende Carbodiimid mit Verbindungen, die mindestens zwei gegenüber Isocyanaten reaktive Gruppen aufweisen, zu substituierten, beispielsweise Urethan- und/oder Harnstoffstrukturen aufweisenden Carbodiimiden umsetzt. Das molare Verhältnis der NCO-Gruppen des Isocyanatgruppen-aufweisenden Carbodiimides zu den gegenüber den Isocyanaten reaktiven Gruppen beträgt üblicherweise 10:1 bis 0,2:1, bevorzugt 5:1 bis 0,5:1.

Alternativ können die erfindungsgemäßen Carbodiimide dadurch erhalten werden, daß man 1,3-Bis-(1-methyl-1-isocyanato-ethyl)benzol, dessen Isocyanatgruppen nicht an aromatische Kohlenstoffatome gebunden sind, mit Verbindungen, die mindestens zwei gegenüber Isocyanaten reaktive Gruppen aufweisen, umsetzt, wobei das Verhältnis von eingesetzten Isocyanatgruppen zu gegenüber Isocyanaten reaktiven Gruppen zwischen 100:1 bis 2:1, bevorzugt 50:1 bis 2:1 beträgt, und anschließend das Isocyanatgruppen aufweisende Reaktionsprodukt in Gegenwart von Katalysatoren unter Kohlendioxidfreisetzung zu Urethan- und/oder Harnstoffstrukturen enthaltenden Carbodiimiden umsetzt. Nach dieser Verfahrensvariante werden zunächst bis zu 50 Gew.-%, vorzugsweise bis zu 23 Gew.-% der Isocyanatgruppen des Diisocyanates mit den gegenüber Isocyanaten reaktiven Verbindungen umgesetzt und danach die freien Isocyanatgruppen in Gegenwart von Katalysatoren unter Kohlendioxidfreisetung ganz oder teilweise zu Carbodiimiden und/oder oligomeren Polycarbodiimiden kondensiert.

Bevorzugt führt man erst die Umsetzung zu den Carbodiimiden durch und setzt anschließend das Isocyanatgruppen-aufweisende Carbodiimid mit den gegenüber Isocyanaten reaktiven Verbindungen um.

Der Verfahrensschritt (1) zur Herstellung der erfindungsgemäßen Carbodiimide durch Umsetzung von Diisocyanaten kann bei erhöhten Temperaturen, z.B. bei Temperaturen von 50 bis 200°C, vorzugsweise von 150 bis 185°C, zweckmäßigerweise in Gegenwart von Katalysatoren unter Kohlendioxidabspaltung durchgeführt werden. Hierfür geeignete Verfahren werden beispielsweise beschrieben in der GB-A-1 083 410, der DE-B 1 130 594 (GB-A-851 936) und der DE-A-11 56 401 (US-A-3 502 722). Als Katalysatoren vorzüglich bewährt haben sich z.B. Phosphorverbindungen, die vorzugsweise ausgewählt werden aus der Gruppe der Phospholene, Phospholenoxide, Phospholidine und Phospholinoxide. Wenn die Reaktionsmischung den gewünschten Gehalt an NCO-Gruppen, entsprechend einem Kondensationsgrad von bevorzugt mindestens 4 besitzt, wird die Polycarbodiimidbildung üblicherweise beendet. Hierzu können die Katalysatoren unter vermindertem Druck abdestilliert oder durch Zusatz eines Desaktivators, wie z.B. Phosphortrichlorid, desaktiviert werden. Die Polycarbodiimidherstellung kann ferner in Abwesenheit oder Gegenwart von unter den Reaktionsbedingungen inerten Lösungsmitteln durchgeführt werden.

Durch geeignete Wahl der Reaktionsbedingungen wie z.B. der Reaktionstemperatur, der Katalysatorart und der Katalysatormenge sowie der Reaktionszeit kann der Fachmann in der üblichen Weise den Kondensationsgrad einstellen. Der Verlauf der Reaktion kann am einfachsten durch Bestimmung des NCO-Gehaltes verfolgt werden. Auch andere Parameter wie z.B. Viskositätsanstieg, Farbvertiefung oder CO₂-Entwicklung kann man für die Verfolgung des Ablaufs und die Steuerung der Reaktion heranziehen.

Als Diisocyanate setzt man 1,3-Bis-(1-methyl-1-isocyanatoethyl)-benzol, im Folgenden auch als TMXDI bezeichnet, ein. Es können auch Gemische mit 1,3-Bis-(1-methyl-1- isocyanato-ethyl)-benzol verwendet werden. In diesem Fall werden bevorzugt mindestens 30 mol% an 1,3-Bis-(1-methyl-1- isocyanato ethyl)-benzol mitverwendet. Besonders bevorzugt weisen die erfindungsgemäßen Carbodiimide mindestens eine der folgenden Struktureinheiten auf, die die Carbodiimidstruktur des besonders bevorzugt einzusetzenden Diisocyanats darstellt:

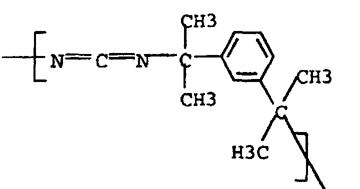

Beispielsweise können die erfindungsgemäßen Carbodiimide folgende Struktur aufweisen: 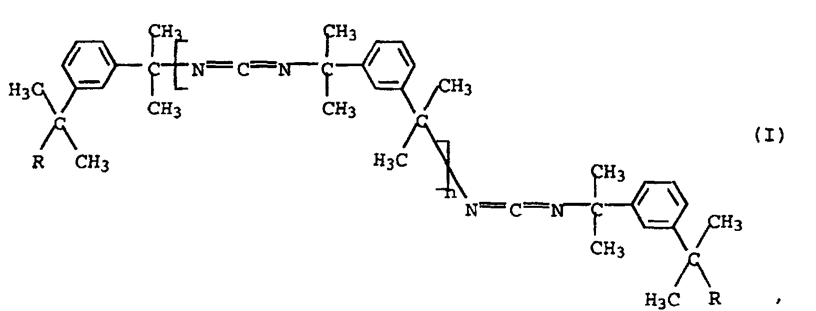 in der
- R: gleich oder verschieden, beispielsweise -NHCONHR¹- oder -NHCOOR¹-Reste,
wobei sich R¹ und R² aus den gegenüber Isocyanaten reaktiven Verbindungen, die im Folgenden beispielhaft beschrieben werden, ergeben und die die abgebildete Struktur mit weiteren Carbodiimidstrukturen verbinden können,
- n: beispielsweise 0, 1, 2, 3, 4, 5, 6, 7, 8, 9, 10 bedeuten können, wobei n wie angegeben ganzzahlig oder - im statistischen Mittel - gebrochen sein kann.

Die erfindungsgemäßen bei einer Temperatur von 25 °C festen Carbodiimide sind somit vom 1,3-Bis-(1-methyl-1-isocyanato-ethyl)-benzol (TMXDI) abgeleitet.

Als gegenüber Isocyanaten reaktiven Verbindungen, die mindestens zwei gegenüber Isocyanaten reaktive Gruppen aufweisen, können zur Herstellung der erfindungsgemäßen Carbodiimide allgemein übliche Substanzen eingesetzt werden, die durch ihre Reaktion mit Isocyanaten Urethan- und/oder Harnstoffgruppen erzeugen. Beispielsweise können aromatische, aliphatische und/oder araliphatische Verbindungen mit 2 bis 20 Kohlenstoffatomen verwendet werden, die Hydroxyl- und/oder Amingruppen als gegenüber Isocyanaten reaktive Gruppen enthalten. Bevorzugt werden als Verbindungen, die mindestens zwei gegenüber Isocyanaten reaktive Gruppen aufweisen, organische Verbindungen mit mindestens zwei Hydroxylgruppen, mit mindestens zwei Amingruppen und/oder mindestens einer Hydroxyl-gruppe und mindestens einer Amingruppe eingesetzt. Beispielsweise können verwendet werden: aromatische, araliphatische und/oder aliphatische Polyole mit 2 bis 20 Kohlenstoffatomen, bevorzugt solche mit primären Hydroxylgruppen. Z.B. können genannt werden: Ethandiol-1,2, Propandiol-1,3, Propandiol-1,2, Butandiol-1,4, -2,4, und/oder -2,3, Pentandiol-1,5, Hexandiol-1,6, Heptandiol-1,7, Oktandiol-1,8, Decandiol-1,10, Neopentylglykol, 2-Methylpropandiol-1,3, 2- und 3-Methylpentandiol-1,5, die Isomeren des Bis(hydroxy-methyl- oder -ethyl)benzols, Hydroxyalkylether von Dihydroxybenzolen, Trimethylolpropan, Glycerin, Pentaerythrit und/oder -Zucker mit beispielsweise 4, 5 oder 6 Hydroxylgruppen.

Weiterhin verwendbar als Verbindungen mit mindestens zwei Hydroxylgruppen sind Polyester- und Polyetheralkohole, wie sie z.B. zur Herstellung von Polyurethanen geeignet sind. Solche Makroole sind z.B. in "Kunststoffhandbuch, Bd. 7: Polyurethane" beschrieben.

Besonders geeignet sind die Makroole, die auch zum Aufbau des gegen Hydrolyse zu schützenden Polymeren verwendet worden sind.

Demgemäß sind zum Hydrolyseschutz von Polyestern besonders Makroole geeignet, die einen Ausschnitt aus der Struktur des Polyesters darstellen. Insbesondere sind hier oligomere Polyethylen- und -butylenterephthalate zu nennen.

Diese Oligomere können erhalten werden entweder aus Ethan- bzw. Butandiol und Terephthalsäure oder niederen Estern der Terephthalsäure durch die allgemein bekannten Kondensationsreaktionen oder aber durch glykolytischen Abbau handelsüblicher hochmolekularer Terephthalester.

Die Oligomere weisen ein Molekulargewicht Mₙ von 260 bis 10000 g/mol, bevorzugt von 260 bis 5000 g/mol, besonders bevorzugt von 350 bis 2000 g/mol auf.

Als Amine sind Amine mit mindestens zwei primären und/oder sekundären Amingruppen zu verwenden. Beispielhaft sind zu nennen: Amine des Molekulargewichtsbereiches von 32 bis 500 g/mol, vorzugsweise von 60 bis 300 g/mol, welche mindestens zwei primäre, mindestens zwei sekundäre oder eine primäre und eine sekundäre Aminogruppe enthalten. Beispiele hierfür sind Diamine wie Diaminoethan, Diaminopropan, Diaminobutan, Diaminopentan, Diaminohexan, Piperazin, 2,5-Dimethylpiperazin, 1-Amino-3,3,5-trimethyl-5-aminomethyl-cyclohexan (Isophorondiamin, IPDA), 4,4'-Diaminodicyclohexylmethan, 1,4-Diaminocyclohexan, Aminoethylethanolamin, Hydrazin, Hydrazinhydrat und/oder Triamine wie Diethylentriamin, und/oder 1,8-Diamino-4-aminomethyloktan.

Weiterhin können Amine verwendet werden, die sich von den genannten Aminen dadurch ableiten, daß eine oder mehrere primäre Amingruppen durch weitere Substituenten wie z.B. Alkylgruppen zu sekundären Amingruppen substituiert werden. Des weiteren können auch Verbindungen, die sowohl mindestens eine Hydroxylgruppe als auch mindestens eine Amingruppe aufweisen, eingesetzt werden, beispielsweise Ethanolamin, Propanolamin, Isopropanolamin, Aminoethylethanolamin oder davon abgeleitete N-Alkylamine.

Bevorzugt werden lineare Alkohole, Amine oder Aminoalkohole, besonders bevorzugt solche mit einer geraden Zahl von C-Atomen verwendet. Weiterhin bevorzugt sind Alkohole, Amine oder Aminoalkohole mit cyclischen Strukturelementen.

Gegebenenfalls kann es zweckmäßig sein, zusätzlich zu den beschriebenen gegenüber Isocyanaten reaktiven Verbindungen mit mindestens zwei funktionellen Gruppen auch monofunktionelle Verbindungen einzusetzen, um das Molekulargewicht der erfindungsgemäßen Carbodiimide zu regulieren, insbesondere wenn die Diisocyanate in einem ersten Schritt zu den Carbodiimiden umgesetzt werden und anschließend die Reaktion der Isocyanatgruppen-enthaltenden Carbodiimide mit den gegenüber Isocyanaten reaktiven Verbindungen erfolgt. Als monofunktionelle gegenüber Isocyanaten reaktive Verbindungen können beispielsweise Amine und bevorzugt Alkohole verwendet werden. Geeignete Amine, z.B. primäre oder vorzugsweise sekundäre Amine, besitzen vorteilhafterweise 1 bis 12 C-Atome, vorzugsweise 2 bis 8 C-Atome. Beispielhaft genannt seien Methyl-, Ethyl-, Propyl-, Butyl-, Pentyl-, Hexyl-, 2-Ethyl-hexyl-, Octyl-, Decyl-, Diethyl-, Dipropyl-, Dibutyl-, Methylbutyl-, Ethylbutyl- und Ethylhexylamin sowie Cyclohexyl- und Benzylamin. Zur Absättigung der Isocyanatgruppen finden jedoch vorzugsweise Alkohole, z.B. primäre oder sekundäre Alkohole mit 1 bis 18 C-Atomen, vorzugsweise 2 bis 8 C-Atomen Verwendung. Als primäre oder sekundäre Alkohole seien beispielhaft genannt: Methanol, Ethanol, n-Propanol, Isopropanol, n-Butanol, sekundärButanol, n-Pentanol, technische Pentanolmischungen, n-Hexanol, technische Hexanolgemische, 2-Ethylhexanol, Octanol, 2-Ethyloctanol, Decanol und Dodecanol sowie Cyclohexanol und Benzylalkohol.

Die erfindungsgemäßen Carbodiimide liegen bei Raumtemperatur, d.h. bei 25°C, bevorzugt bei 30°C, besonders bevorzugt bei 40°C, insbesondere bei 50°C und einem Druck von 1013 mbar als feste Substanzen vor. Dadurch weisen die Carbodiimide den Vorteil auf, daß sie mit bekannten Verfahren granuliert oder gemahlen werden können und als Feststoff in üblichen Apparaturen der "Kunststoffverarbeitenden Industrie bei der Verarbeitung oder Herstellung von Kunststoffen einsetzbar sind. Dieser Einsatz von Feststoffen ist in üblichen Apparaturen gegenüber dem Einsatz von Flüssigkeiten wesentlich einfacher zu handhaben.
Die erfindungsgemäßen Carbodiimide enthaltend bevorzugt mindestens 4 Carbodiimidstrukturen, bevorzugt mehr als vier Carbodiimidstrukturen, besonders bevorzugt beträgt der mittlere Kondensationsgrad (Zahlenmittelwert), d.h. die mittlere Anzahl an Carbodiimidstrukturen in den erfindungsgemäßen Carbodiimiden 4,1 bis 20, insbesondere 5 bis 15. Des weiteren enthalten die erfindungsgemäßen Verbindungen Urethan- und/oder Harnstoffstrukturen, die durch die Umsetzung von Isocyanatgruppen der bei der Herstellung eingesetzten Diisocyanate mit gegenüber Isocyanaten reaktiven Verbindungen, beispielsweise hydroxylgruppenhaltigen Verbindungen und/oder Aminen, entstehen.

Die Carbodiimidstrukturen der erfindungsgemäßen Verbindungen sind an nicht-aromatische Kohlenwasserstoffe gebunden. Dies bietet den wesentlichen Vorteil, daß bei einer Spaltung der Carbodiimide keine aromatischen Amine freigesetzt werden und somit die erfindungsgemäßen Carbodiimide toxikologisch wesentlich weniger bedenklich sind als die beispielsweise in EP-A 460 481 beschriebenen Carbodiimide.

Aus den erfindungsgemäßen Carbodiimiden und oligomeren Polycarbodiimiden entstehen bei der Reaktion mit Carbonsäuren und/oder carboxylgruppenhaltigen Verbindungen im Falle von Carbodiimiden auf der Basis von TMXDI araliphatische Isocyanate mit einer im Vergleich zu aromatischen Isocyanaten geringen Reaktivität. Die gebildeten araliphatischen Isocyanate beeinflussen daher beispielsweise die Kennzahl einer Polyadditionsreaktion bei einer Urethanbildung praktisch nicht. Folglich sind die Molekulargewichte der gebildeten Polyurethane und damit verbunden ihre mechanischen Eigenschaften konstant und sehr gut reproduzierbar.

Die erfindungsgemäßen Carbodiimide besitzen an einen nicht-aromatischen Kohlenstoff gebundene, bevorzugt sterisch gehinderte Isocyanat-, Harnstoff- und/oder Urethangruppen, zeigen eine mit den technisch genutzten aromatischen Carbodiimiden und aromatischen Polycarbodiimiden zumindest vergleichbare hohe Hydrolyseschutzwirkung und Lichtbeständigkeit und können unter Beachtung der Arbeitsschutzvorschriften problemlos in die Estergruppen enthaltenden Polykondensaticns- und Polyadditionsprodukte eingebracht werden. Vorteilhaft ist ferner die große Anzahl an wirksamen Carbodiimidgruppen, bezogen auf das Molekulargewicht der Carbodiimide. Die Carbodiimide sind mit den Estergruppen enthaltenden Polyadditions- und Polykondensationsprodukten, insbesondere mit Polyesterurethankautschuken, gut verträglich und auch problemlos mit diesen Materialien in der Schmelze homogen mischbar.

Die erfindungsgemäßen Monocarbodiimide und/oder oligomeren Polycarbodiimide eignen sich hervorragend als Akzeptor für Carboxylverbindungen und finden daher vorzugsweise Verwendung als Stabilisatoren gegen den hydrolytischen Abbau von Estergruppen enthaltenden Verbindungen, beispielsweise Estergruppen enthaltende Polymere, z.B.Polykondensationsprodukte wie beispielsweise thermoplastische Polyester wie Polyethylen- und -butylenterephthalat, Polyetherester, Polyesterester, Polyamide, Polyesteramide, Polycaprolactone sowie ungesättigte Polyesterharze und z.B. Blockcopolymere aus Polyethylen- oder butylenterephthalat, Polycaprolacton und Polyadditionsprodukte, z.B. Polyurethane, Polyharnstoffe und Polyurethan-Polyharnstoff-Elastomere, die Estergruppen enthalten. Diese Estergruppen enthaltenden Verbindungen sind allgemein bekannt. Ihre Ausgangssubstanzen, Herstellverfahren, Strukturen und Eigenschaften sind in der Standardliteratur vielfältig beschrieben. Aufgrund der guten Löslichkeit in den Aufbaukomponenten zur Herstellung von Polyurethanen und der guten Verträglichkeit mit den gebildeten Polyurethanen eignen sich die erfindungsgemäßen (Poly)carbodiimide insbesondere als Stabilisatoren gegen den hydrolytischen Abbau von Polyurethanen, vorzugsweise kompakten oder zelligen Polyurethan-Elastomeren und insbesondere thermoplastischen Polyurethanen.

Weisen die erfindungsgemäßen Carbodiimide endständige Isocyanatgruppen auf, beispielsweise wenn ein Isocyanatgruppen aufweisendes Carbodiimid mit einem Unterschuß an gegenüber Isocyanaten reaktiven Gruppen eingesetzt wird, können die Carbodiimide bei der Herstellung der Polyadditionsprodukte durch Umsetzung von Isocyanaten mit gegenüber Isocyanaten reaktiven Verbindungen eingesetzt werden.

Die Konzentration der erfindungsgemäßen Carbodiimide in den zu stabilisierenden Estergruppen enthaltenden Polykondensationsoder Polyadditionsprodukte beträgt im allgemeinen 0,05 bis 10 Gew.-%, vorzugsweise 0,1 bis 5 Gew.-%, bezogen auf das Gesamtgewicht der Mischung. In Einzelfällen kann, je nach der Beanspruchung des Kunststoffs durch Hydrolyse, die Konzentration auch höher sein.

Die erfindungsgemäß verwendbaren Carbodiimide können nach verschiedenen Methoden in die zu stabilisierenden Estergruppen enthaltenden Produkte eingebracht werden. Beispielsweise können die erfindungsgemäßen Carbodiimide mit einer der Aufbaukomponenten zur Herstellung der Polyadditionsprodukte, z.B. den Polyisocyanaten oder/und Polyhydroxylverbindungen zur Herstellung von Polyurethanen, gemischt werden oder die Carbodiimide können der Reaktionsmischung zur Herstellung der Polyurethane zudosiert werden. Nach einer anderen Verfahrensweise können die erfindungsgemäßen Carbodiimide der Schmelze der ausreagierten Polyadditions- oder Polykondensationsprodukte einverleibt werden. Es ist jedoch auch möglich, Granulate der Polyadditions- oder Polykondensationsprodukte mit den erfindungsgemäßen Carbodiimiden zu beschichten oder mit den pulverisierten, pelletierten oder granulierten erfindungsgemäßen Carbodiimiden zu mischen und bei einer nachfolgenden Herstellung von Formkörpern durch Schmelzextrusion in die Kunststoffmassen einzubringen. Zur Herstellung von Polyurethan-Gießelastomeren und TPU auf Polyesterbasis werden nach einer bevorzugten Ausführungsform zunächst die carboxylgruppenhaltigen Polyester-polyole zur Reduzierung der Säuregehalte mit den erfindungsgemäßen Carbodiimiden behandelt und danach diese, gegebenenfalls unter Zugabe von weiteren Mengen an Carbodiimiden, mit Polyisocyanaten, gegebenenfalls in Gegenwart zusätzlicher Hilfsmittel und Additive, zur Reaktion gebracht. Weiter können die erfindungsgemäßen Carbodiimide über die Isocyanat-Komponente in das Polyurethan eingebracht werden. Besonders zum Tragen kommen die erfindungsgemäßen Carbodiimide jedoch dann, wenn sie während der üblichen Konfektionierung in das estergruppenhaltige Polymer eingebracht werden.

Neben der Wirksamkeit als Stabilisator gegen den hydrolytischen Abbau von Estergruppen enthaltenden Polyadditions- oder Polykondensationsprodukten oder zur Entsäuerung von Polyesterolen, welche zur Herstellung von polyesterhaltigen Kunststoffen, insbesondere Polyurethankautschuken, verwendet werden können, eignen sich die Carbodiimide z.B. auch zum Abbruch von Veresterungsreaktionen bei der Herstellung von Polyestern, wenn der gewünschte Polykondensationsgrad erreicht ist.

### Beispiele

750 Gew.-Teile (3,1 mol) 1,3-Bis-(1-methyl-1-isocyanato-ethyl)-benzol mit einem NCO-Gehalt von 34,4 Gew.-% wurden in Gegenwart von 1,5 Gew.-Teilen, bezogen auf das Isocyanat, 1-Methyl-2-phospholen-1-oxid lösungsmittelfrei auf 180°C erhitzt und bei dieser Temperatur unter mäßiger Kohlendioxidentwicklung kondensiert. Nach Erreichen eines NCO-Gehalts der Reaktionsmischung von 11,2 Gew.-% wurden der zugesetzte Katalysator und Reste von nicht umgesetztem 1,3-Bis-(1-methyl-1-isocyanato-ethyl)-benzol bei einer Temperatur von 180°C und unter einem Druck von 1 mbar abdestilliert.

Man erhielt 570 Gew.-Teile einer Mischung von Carbodiimiden mit einem NCO-Gehalt von 7,79 Gew.-%, einem Gehalt an -N=C=N--Gruppen von 15,2 Gew.-% (berechnet), einem Schmelzpunkt < 30°C und einer Jodfarbzahl von 5 bis 7, gemessen nach DIN 6162.

Die Struktur der Isocyanatgruppen aufweisenden Mischung aus Mono- und oligomeren Polycarbodiimiden wurde durch IR-Spektrum und gelchromatographische Analyse nachgewiesen.

Diese Mischung von Carbodiimiden wird im Folgenden als Carbodiimid 1 bezeichnet.

### Beispiele 1 bis 12

500 g dieser Mischung Carbodiimid 1 wurden in einem Rührkolben mit den in der Tabelle 1 angegebenen Alkoholen bei 140°C umgesetzt, bis der angegebene NCO-Gehalt erreicht war. Das in Tabelle 1 angegebene Molverhältnis stellt das eingesetzte Molverhältnis von Isocyanatgruppen:Diolen:Monoolen dar.

**Tabelle 1**

| Beispiel | Diol | Monool | Molverhältnis | NCO-Gehalt [Gew.-%] |
|---|---|---|---|---|
| 1 | Ethan-1,2-diol | - | 1:0,5:0 | 0,08 |
| 2 | Ethan-1,2-diol | Ethanol | 1:0,45:0,1 | 0,17 |
| 3 | Ethan-1,2-diol, Butan-1,4-diol (1:1) | - | 1:0,5:0 | 0,08 |
| 4 | Butan-1,4-diol | - | 1:0,5:0 | 0,07 |
| 5 | Butan-1,4-diol | Ethanol | 1:0,35:0,3 | 0,05 |
| 6 | Neopentylglykol | - | 1:0,5:0 | 0,38 |
| 7 | Neopentylglykol | - | 1:0,55:0 | 0,01 |
| 8 | HQEE | - | 1:0,5:0 | 0,02 |
| 9 | CHDM | - | 1:0,5:0 | 0,01 |
| 10 | HMBA | - | 1:0,5:0 | 0,08 |
| 11 | Trimethylolpropan | - | 1:0,6:0 | 0,05 |
| 12 | Hexan-1,6-diol | - | 1:0,5:0 | 0,2 |

| | | | | |
|---|---|---|---|---|
| HQEE: Hydrochinon-bis-(2-hydroxyethyl)ether | | | | |
| CHDM: Cyclohexan-1,4-dimethanol | | | | |
| HMBA: 1,4-Di(hydroxymethyl)benzol | | | | |

### Beispiele 13 bis 20

500 g dieser Mischung Carbodiimid 1 wurden in einem Rührkolben mit den in der Tabelle 2 angegebenen Aminen und gegebenenfalls Alkoholen bei 140°C umgesetzt, bis der angegebene NCO-Gehalt erreicht war. Das in Tabelle 2 angegebene Molverhältnis stellt das eingesetzte Molverhältnis von Isocyanatgruppen: Diaminen: Alkoholen dar.

**Tabelle 2**

| Beispiel | Diamin | Alkohol | Molverhältnis | NCO-Gehalt [Gew.-%] |
|---|---|---|---|---|
| 13 | Ethan-1,2-diamin | - | 1:0,5:0 | 0,00 |
| 14 | Isophorondiamin | Ethanol | 1:0,25:0,5 | 0,01 |
| 15 | Ethan-1,2-diamin | Ethan-1, 2-diol | 1:0,13:0,38 | 0,09 |
| 16 | Ethan-1,2-diamin | Ethan-1, 2-diol | 1:0,25:0,25 | 0,06 |
| 17 | Ethan-1,2-diamin | Ethan-1, 2-diol | 1:0,375:0,125 | 0,06 |
| 18 | Hexan-1,6-diamin | - | 1:0,5:0 | 0 |
| 19 | Butan-1,4-diamin | - | 1:0,5:0 | 0,01 |
| 20 | Isophorondiamin | - | 1:0,5:0 | 0,01 |

| | | | | |
|---|---|---|---|---|
| Isophorondiamin: 1-Amino-3,3,5-trimethyl-5-aminomethyl-cyclohexan | | | | |

Alle in den Beispielen 1 bis 20 hergestellten Carbodiimide wiesen Jodfarbzahlen kleiner als 8, in der Regel kleiner als 5 auf. Die Carbodiimide konnten durch Mahlen in einer Schlagkreuzmühle ohne weiteres zu feinen, rieselfähigen, nicht klumpenden Pulvern verarbeitet werden.

Die pulverisierten Carbodiimide wurden während 14 Tagen in einem Wärmeschrank bei 35°C gelagert. Nach dieser Zeit waren sie immer noch gut rieselfähig; einige kleinere Klumpen waren leicht zu zerdrücken.

### Beispiele 21 bis 41

Die erfindungsgemäßen Carbodiimide der Beispiele 1 bis 20 wurden in einer Konzentration von 0,65 Gew.-%, bezogen auf das Gewicht des TPUs auf einem Zweischneckenextruder mit Feststoffdosierung in ein thermoplastisches Polyurethan (TPU) eingearbeitet, das erhalten wurde durch Umsetzung von
440 g (1, 76 mol) 4,4'-Diphenylmethandiisocyanat
1000 g (0,5 mol) 1,4-Butandiol-1,6-Hexandiol-Polyadipat
113 g (1,26 mol) 1,4-Butandiol.

Aus dem Carbodiimid-haltigen Granulat wurden mit Hilfe des Spritzgußverfahrens Prüfkörper hergestellt.

An den Prüfkörpern wurde unmittelbar nach ihrer Herstellung und nach einer 21tägigen Lagerung bei 80°C in Wasser die Zugfestigkeit und die Bruchdehnung nach DIN 53 504 gemessen.

Die verwendeten Carbodiimide und die gemessenen mechanischen Eigenschaften sind in Tabelle 3 zusammengefaßt.

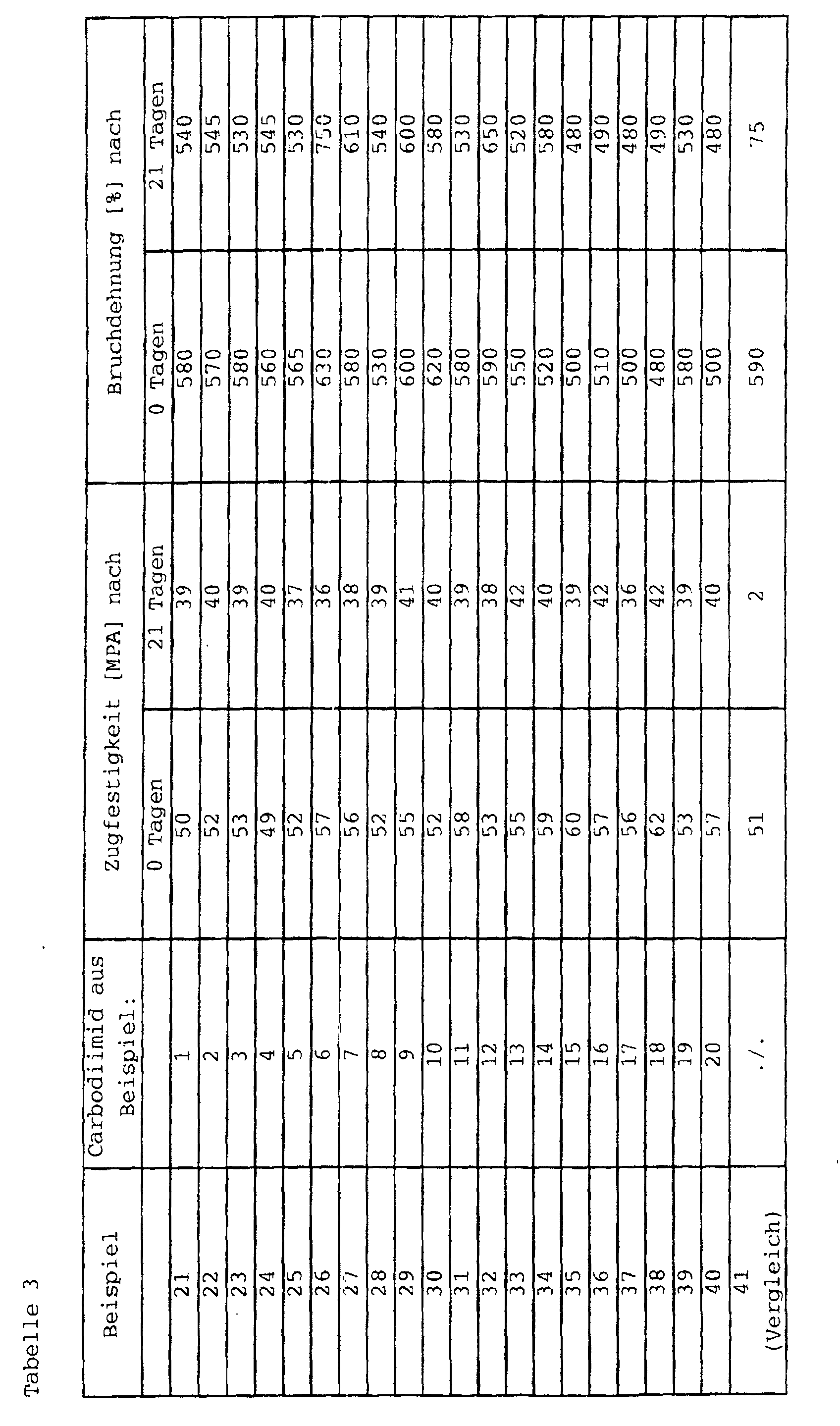

### Beispiel 42

616 g (0,947 mol) eines durch Umesterung aus Dimethylterephthalat und Ethandiol-1,2 gewonnenes PET-Oligomeren der OH-Zahl 172 und 500 g (0,464 mol) des Polycarbodiimids 1 wurden in einem Rührkolben bei 200°C umgesetzt, bis ein NCO-Gehalt von < 0,3 Gew.-% erreicht war.

Das Produkt wies eine Glastemperatur von 45°C auf.

### Beispiel 43

Beispiel 1 wurde wiederholt, jedoch wurden 454 g Oligo-PET verwendet.

Das Produkt wies eine Glastemperatur von 57°C auf.

### Beispiel 44

Beispiel 1 wurde wiederholt, jedoch wurden 404 g Oligo-PET verwendet.

Das Produkt wies eine Glastemperatur von 57°C auf.

### Beispiel 45

495 g (0,932 mol) eines durch Umesterung aus Dimethylterephthalat und Butandiol-1,4 gewonnenes PBT-Oligomeren der OH-Zahl 211 und 500 g (0,464 mol) eines Polycarbodiimide mit einem NCO-Gehalt von 7,79 Gew.-%, das nach der Lehre der DOS 4 318 979 hergestellt war, wurden in einem Rührkolben bei 200°C umgesetzt, bis ein NCO-Gehalt von < 0,3 Gew.-% erreicht war.

### Beispiel 46

Beispiel 4 wurde wiederholt, jedoch wurden 273 g Oligo-PBT verwendet.

### Beispiel 47

Beispiel 4 wurde wiederholt, jedoch wurden 330 g Oligo-PBT verwendet.

## Patentansprüche

1. Verfahren zur Herstellung von bei Raumtemperatur festen Carbodiimiden enthaltend Carbodiimidstrukturen sowie Urethanund/oder Harnstoffstrukturen, **dadurch gekennzeichnet, daß** man 1,3-Bis-(1-methyl-1-isocyanato-ethyl)benzol, dessen Isocyanatgruppen nicht an aromatische Kohlenstoffatome gebunden sind, in Gegenwart von Katalysatoren unter Kohlendioxidab-spaltung zu Carbodiimiden umsetzt und anschließend das Isocyanatgruppen-aufweisende Carbodiimid mit Verbindungen, die mindestens zwei gegenüber Isocyanaten reaktive Gruppen aufweisen, umsetzt.

2. Verfahren zur Herstellung von bei Raumtemperatur festen Carbodiimiden enthaltend Carbodiimidstrukturen sowie Urethanund/oder Harnstoffstrukturen, **dadurch gekennzeichnet, daß** man 1,3-Bis-(1-methyl-1-isocyanato-ethyl)benzol, dessen Isocyanatgruppen nicht an aromatische Kohlenstoffatome gebunden sind, mit Verbindungen, die mindestens zwei gegenüber Isocyanaten reaktive Gruppen aufweisen, umsetzt, wobei das Verhältnis von eingesetzten Isocyanatgruppen zu gegenüber Isocyanaten reaktiven Gruppen mindestens 2 : 1 beträgt, und anschließend das Isocyanatgruppen aufweisende Reaktionsprodukt in Gegenwart von Katalysatoren unter Kohlendioxidfreisetzung zu Carbodiimiden umsetzt.

3. Verfahren nach Anspruch 1 oder 2, **dadurch gekennzeichnet, daß** man als Verbindungen, die mindestens zwei gegenüber Isocyanaten reaktive Gruppen aufweisen, organische Verbindungen mit mindestens zwei Hydroxylgruppen, mit mindestens zwei Amingruppen und/oder mit mindestens einer Hydroxylgruppe und mindestens einer Amingruppe einsetzt.

4. Verfahren nach einem der Ansprüche 1 oder 2, **dadurch gekennzeichnet, daß** die Carbodiimide mindestens 4 Carbodiimidstrukturen aufweisen.

5. Bei Raumtemperatur feste Carbodiimide erhältlich durch ein Verfahren gemäß Anspruch 1 oder 2.

6. Bei Raumtemperatur feste Carbodiimide gemäß Anspruch 5, **dadurch gekennzeichnet, daß** diese eine Struktur gemäß der Formel (I) aufweisen 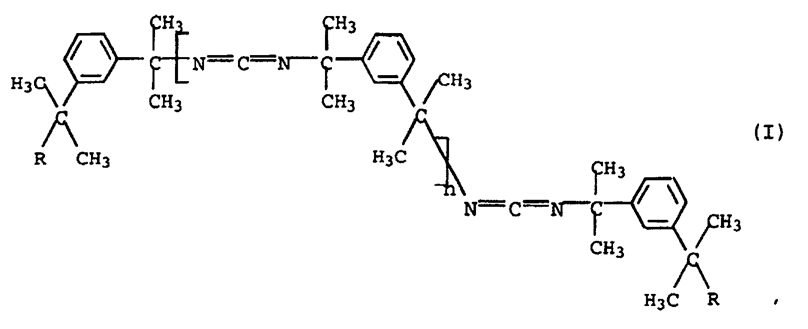 in der
R gleich oder verschieden -NHCONHR¹- oder -NHCOOR¹-Reste bedeutet,
wobei R¹ einen aromatischen, aliphatischen und/oder araliphatischen Rest mit 2 bis 20 Kohlenstoffatomen, der mindestens eine Hydroxylgruppe oder Amingruppe enthält:, darstellt, und
n eine Zahl von 0 bis 10, die ganzzahlig oder im statistischen Mittel gebrochen sein kann, bedeutet.

7. Mischungen enthaltend Verbindungen, die Esterstrukturen aufweisen, und Carbodiimide gemäß einem der Ansprüche 5 oder 6.

8. Mischungen enthaltend Carbodiimide gemäß Anspruch 5 oder 6 und mindestens eine Verbindung aus der folgenden Gruppe: Polyurethane, die Esterstrukturen aufweisen, Polykondensationsprodukte wie thermoplastische Polyester wie Polyethylen und -butylenterephthalat, Polyetherester, Polyesterester, Polyesteramide, Polycaprolactone, ungesättigte Polyesterharze und Polyamide.

9. Mischungen gemäß Anspruch 7 oder 8 enthaltend Carbodiimide gemäß Anspruch 5 oder 6 in einer Menge von 0,05 bis 10 Gew.-%, bezogen auf das Gesamtgewicht der Mischung.

10. Verwendung von Carbodiimiden gemäß Anspruch 5 oder 6 als Stabilisatoren gegen den hydrolytischen Abbau von Estergruppen enthaltenden Verbindungen.

## Claims

1. A process for preparing carbodiimides which are solid at room temperature and contain carbodiimide structures and also urethane and/or urea structures, which comprises converting 1,3-bis(1-methyl-1-isocyanatoethyl)benzene, whose isocyanate groups are not bonded to aromatic carbon atoms, to carbodiimides in the presence of catalysts with carbon dioxide elimination and then reacting the carbodiimide having isocyanate groups with compounds which have at least two groups reactive toward isocyanates.

2. A process for preparing carbodiimides which are solid at room temperature and contain carbodiimide structures and also urethane and/or urea structures, which comprises reacting 1,3-bis(1-methyl-1-isocyanatoethyl)benzene, whose isocyanate groups are not bonded to aromatic carbon atoms, with compounds which have at least two groups reactive toward isocyanates where the ratio of isocyanate groups used to groups reactive toward isocyanates is at least 2 : 1, and then converting the reaction product having isocyanate groups to carbodiimides in the presence of catalysts with release of carbon dioxide.

3. A process as claimed in claim 1 or 2, wherein the compounds used which have at least two groups reactive toward isocyanates are organic compounds having at least two hydroxyl groups, having at least two amine groups and/or having at least one hydroxyl group and at least one amine group.

4. A process as claimed in claim 1 or 2, wherein the carbodiimides have at least 4 carbodiimide structures.

5. A carbodiimide which is solid at room temperature and is obtainable by a process as claimed in claim 1 or 2.

6. A carbodiimide which is solid at room temperature as claimed in claim 5, which has a structure of the formula (I) 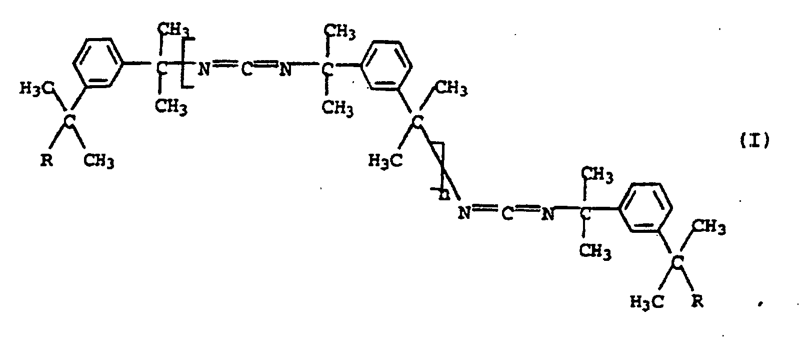 where
R is in each case the same or different and is an -NHCONHR¹- or -NHCOOR¹- radical
where R¹ is an aromatic, aliphatic and/or araliphatic radical having from 2 to 20 carbon atoms which contains at least one hydroxyl group or amine group, and
n is a number from 0 to 10 which may be an integer or may on average be a fraction.

7. A mixture comprising compounds which have ester structures and carbodiimides as claimed in any of claims 5 or 6.

8. A mixture comprising carbodiimides as claimed in claim 5 or 6 and at least one compound from the following group:
polyurethanes which have ester structures, polycondensation products such as thermoplastic polyesters such as polyethylene and -butylene terephthalate, polyether esters, polyester esters, polyesteramides, polycaprolactones, unsaturated polyester resins and polyamides.

9. A mixture as claimed in claim 7 or claim 8 comprising carbodiimides as claimed in claim 5 or 6 in an amount of from 0.05 to 10% by weight, based on the total weight of the mixture.

10. The use of carbodiimides as claimed in claim 5 or 6 as stabilizers against the hydrolytic degradation of compounds containing ester groups.

## Revendications

1. Procédé pour la préparation de carbodiimides solides à température ambiante, contenant des structures de carbodiimide ainsi que des structures d'uréthane et/ou d'urée, **caractérisé en ce qu'**on transforme du 1,3-bis-(1-méthyl-1-isocyanato-éthyl)benzène, dont les groupes isocyanate ne sont pas liés à des atomes de carbone aromatiques, en carbodiimides en présence de catalyseurs avec dissociation de dioxyde de carbone et on transforme ensuite le carbodiimide présentant des groupes isocyanate avec des composés qui présentent au moins deux groupes réactifs par rapport aux isocyanates.

2. Procédé pour la préparation de carbodiimides solides à température ambiante, contenant des structures de carbodiimide ainsi que des structures d'uréthane et/ou d'urée, **caractérisé en ce qu'**on transforme du 1,3-bis-(1-méthyl-1-isocyanato-éthyl)benzène, dont les groupes isocyanate ne sont pas liés à des atomes de carbone aromatiques, avec des composés qui présentent au moins deux groupes réactifs par rapport aux isocyanates, le rapport groupes isocyanate utilisés à groupes réactifs par rapport aux isocyanates étant d'au moins 2:1 et on transforme ensuite le produit de réaction présentant les groupes isocyanate en carbodiimides en présence de catalyseurs avec libération de dioxyde de carbone.

3. Procédé selon la revendication 1 ou 2, **caractérisé en ce qu'**on utilise des composés organiques présentant au moins deux groupes hydroxyle, au moins deux groupes amine et/ou au moins un groupe hydroxyle et au moins un groupe amine comme composés qui présentent au moins deux groupes réactifs par rapport aux isocyanates.

4. Procédé selon l'une quelconque des revendications 1 ou 2, **caractérisé en ce que** les carbodiimides présentent au moins 4 structures de carbodiimide.

5. Carbodiimides solides à température ambiante pouvant être obtenus par un procédé selon la revendication 1 ou 2.

6. Carbodiimides solides à température ambiante selon la revendication 5, **caractérisés en ce qu'**ils présentent une structure selon la formule (I) 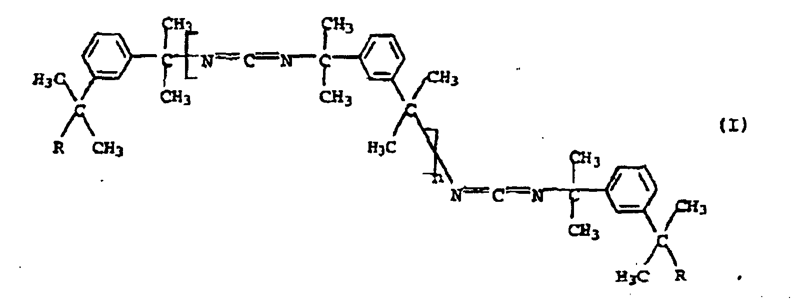 dans laquelle
R est identique ou différent et signifie des radicaux -NHCONHR¹- ou -NHCOOR¹-, R¹ représentant un radical aromatique, aliphatique et/ou araliphatique comprenant 2 à 20 atomes de carbone, qui contient au moins un groupe hydroxyle ou groupe amine et
n signifie un nombre de 0 à 10, qui peut être un nombre entier ou fractionnaire dans la moyenne statistique.

7. Mélanges contenant des composés qui présentent des structures d'ester et des carbodiimides selon l'une quelconque des revendications 5 ou 6.

8. Mélanges contenant des carbodiimides selon la revendication 5 ou 6 et au moins un composé du groupe suivant : les polyuréthanes, qui présentent des structures d'ester, les produits de polycondensation tels que les polyesters thermoplastiques tels que le poly(téréphtalate d'éthylène) et le poly(téréphtalate de butylène), les polyétheresters, les polyesteresters, les polyesteramides, les polycaprolactones, les résines insaturées de polyester et les polyamides.

9. Mélanges selon la revendication 7 ou 8 contenant des carbodiimides selon la revendication 5 ou 6 en une quantité de 0,05 à 10% en poids par rapport au poids total du mélange.

10. Utilisation de carbodiimides selon la revendication 5 ou 6 comme stabilisants contre la dégradation hydrolytique de composés contenant des groupes ester.
